(19) Europäisches Patentamt
European Patent Office
Office européen des brevets

(11) Publication number: 0 193 362 B1

(12) # EUROPEAN PATENT SPECIFICATION

(45) Date of publication of the patent specification: 20.09.89

(51) Int. Cl.[4]: C07C 121/48, H01B 1/12, H01L 29/28 // G01K7/22

(21) Application number: 86301250.6

(22) Date of filing: 21.02.86

(54) **Organic compounds of 7,7,8,8-tetracyanoquinodimethane and method for preparing the same.**

(30) Priority: 23.02.85 JP 35042/85

(43) Date of publication of application: 03.09.86 Bulletin 86/36

(45) Publication of the grant of the patent: 20.09.89 Bulletin 89/38

(84) Designated Contracting States: DE FR GB

(56) References cited:
US-A-3 162 641
US-A-4 229 364

(73) Proprietor: Matsushita Electric Industrial Co., Ltd., 1006, Oaza Kadoma, Kadoma-shi Osaka-fu, 571(JP)

(72) Inventor: Suga, Sadaharu, 8-1-7, Uno, Tamano-shi, Okayama-ken 706(JP)
Inventor: Noguchi, Ayashi, 782, Tsudaka, Okayama-shi, Okayama-ken 701-11(JP)
Inventor: Yasui, Shigeo, 558-2, Shinogoze, Okayama-shi, Okayama-ken 703(JP)

(74) Representative: Spencer, Graham Easdale et al, A.A. Thornton & CO Northumberland House 303-306, High Holborn, London WC1V 7LE(GB)

EP 0 193 362 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

This invention is concerned with certain 7,7,8,8-tetracyanoquinodimethane (hereinafter referred to simply as TCNQ) derivatives, with a method for their preparation, and with compositions containing such compounds.

Integrated circuit technology has recently progressed to a remarkable extent and very large integrated circuits (VLSI) are now being made. However, the technology for miniaturizing integrated circuits is near the limit and different approaches are required.

To this end, various lines of research based on molecular electronics are being pursued.

We have sought organic compounds which have an electrical conductivity substantially equal to that of metals and which are such that their molecules can be arranged regularly in a molecular unit. Organic compounds having these characteristics were not previously known.

Organic compounds having a conductivity which is substantially equal to that of metals, are known, such as a complex of TCNQ and tetrathiofulvalene (hereinafter referred to as TTF); this complex has the formula:

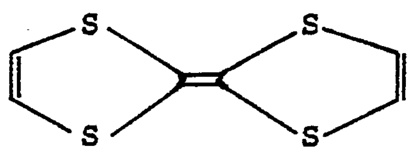 • 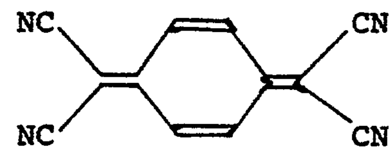

(TTF) (TCNQ)

TCNQ and TTF form a charge transfer complex because TCNQ is a strong electron acceptor and TTF is an electron donor. The complex has an electrical conductivity, the maximum value of which is comparable with the electric conductivity of mercury. However, the complex of TTF and TCNQ cannot be processed or manufactured as a molecular unit.

The Langmuir-Blodgett technique is generally known in the art as a technique for arranging molecules in a monomolecular layer. However, known TCNQ derivatives cannot be processed to form a monomolecular layer of film by the Langmuir-Blodgett technique.

Meanwhile, many studies have been directed to synthesizing TCNQ derivatives. U.S. Patent 3 558 671 discloses a method for making TCNQ fluoride and cyano TCNQ in which quaternary ammonium cations are used to form corresponding salts for dyestuff or pigment use. U.S. Patent 3 739 008 describes a different method of making TCNQ fluoride and cyano TCNQ for use as a dyestuff or antistatic agent. In U.S. Patent 3 687 987, a method of making dialkoxyl TCNQ in which the alkoxy groups contain up to 8 carbon atoms is described. In U.S. Patent 3 526 497, a method for synthesizing dialkoxyl TCNQ and dialkoxyl TCNQ fluoride in which the alkoxy groups contain up to 13 carbon atoms, is described. U.S. Patents 3 115 506 and 3 162 641 describe methods of making TCNQ having an alkyl substituent with up to 8 carbon atoms from an alkylsubstituted 1,4-cyclohexane-dione.

A method for synthesizing TCNQ having an alkyl substituent with up to 3 carbon atoms is described in Journal of Organic Chemistry, Vol. 28, p. 2719, 1963; in this method methyl-para-dimethoxybenzene obtained from 2-methylhydroquinone is reduced.

We have now surprisingly found that TCNQ having an alkyl substituent containing from 10 to 22 carbon atoms, has an electrical conductivity comparable with that of metals and can be arranged in a monomolecular layer by the Langmuir-Blodgett technique.

These compounds are novel and constitute one aspect of the invention.

According to the present invention, therefore, there are provided 7,7,8,8-tetracyanoquinodimethane derivatives of the formula

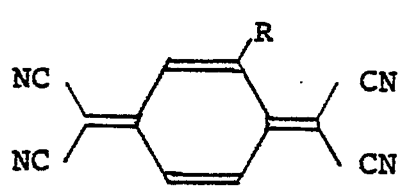 I

in which R is an alkyl group having from 10 to 22 carbon atoms.

The present invention also comprises a charge transfer complex which comprises a 7,7,8,8-tetracyanoquinodimethane derivative of formula I, as an electron acceptor, and at least one of tetrathiofulvalene, heterocyclic quaternary ammonium salts and cyanine dyes, as an electron acceptor.

The present invention further comprises a method of preparing a 7,7,8,8-tetracyanoquinodimethane derivative of formula I, which comprises hydrogenating a hydroquinone derivative containing an alkyl

substituent having from 10 to 22 carbon atoms to form a corresponding alkyl-1,4-cyclohexanedione derivative, reacting the latter with malononitrile to form a corresponding alkyl-tetracyanocyclohexanedimethylidene derivative, and oxidising the latter to form the desired compound.

A TCNQ derivative of formula I may be formed into a monomolecular layer or film by preparing a solution of the derivative in an organic solvent such as chloroform or benzene, the solution optionally also containing a higher fatty acid, such as arachic acid, and allowing drops of the solution to fall on to the surface of water. The monomolecular layer is formed on the surface of the water after the organic phase is evaporated. A monomolecular film or a built-up film can be formed on a substrate by the Langmuir-Blodgett technique.

The monomolecular film or built-up film of the TCNQ derivative obtained in this way forms a charge transfer complex with an electron donor, such as TTF, heterocyclic quaternary ammonium salts, cyanine dyes, etc. The thin films of such charge transfer complexes have conductivities comparable with those of metals.

Such thin films are useful in various devices in the molecular electronics field, such as organic semiconductor devices, light memory devices, and photoelectric devices. For example an N-butylpyridinium TCNQ derivative charge transfer complex has a resistivity-temperature relationship wherein the resistivity varies abruptly near 100°C. This complex can therefore be used as a temperature detector.

In order that the invention may be more fully understood, the following examples are given by way of illustration.

## EXAMPLE 1

(a) 2-Dodecyl-1,4-cycloexanedione

A mixture of 20 g of 1,4-dimethoxy-2-dodecyl benzene, 30 ml of alcohol and 400 ml of diethyl ether was added to liquid ammonia at a temperature of –50°C in a stream of nitrogen. 9.6 g of metallic sodium was added with stirring to initiate the reaction which was then continued for 20 hours. After this time, ammonia was removed and an aqueous solution of ammonium chloride was added. This mixture was adjusted to pH 2–3 by the addition of concentrated hydrochloric acid and was refluxed for 3 hours.

After refluxing, the organic layer was separated from the aqueous layer. The aqueous layer was extracted with ethyl acetate and the extract was combined with the organic layer. The organic layer was washed with water, dried and concentrated. After concentration, the organic layer was purified by silica gel column chromatography, and 2.0 g of 2-dodecyl-1,4-cyclohexane-dione was obtained. This compound had a melting point of 73°C and an IR absorption band at 1710 $cm^{-1}$ which corresponds to a carbonyl group.

(b) 1,4-Bis-(dicyanomethylene)-2-dodecyl-cyclohexane

A mixture of 2 g (0.0071 M) of the thus obtained 2-dodecyl-1,4-cyclohexanedione and 1.4 g (0.0212 M) of malononitrile was heated in a water bath to form a solution. ß-Alanine, as a catalyst, and 2 ml of water were added to the solution and the mixture was stirred at a temperature between 40°C and 50°C for 8 hours in order to effect the desired reaction. After cooling, the reaction mixture was dissolved in chloroform and the resulting solution was washed with water and then dried. The dried solution was concentrated under reduced pressure and purified by silica gel column chromatography. 1.26 g of white crystals of 1,4-bis-(dicyanomethylene)-2-dodecylcyclohexane were obtained. This compound had a melting point of 97°C and an IR absorption band of 2250 $cm^{-1}$ which corresponds to a cyano group.

(c) 2-Dodecyl-7,7,8,8-tetracyanoquinodimethane

2.46 g (0.0065 M) of the thus obtained 1,4-bis(dicyanomethylene)-2-dodecyl cyclohexane were dissolved in 90 ml of dried acetonitrile. 30 ml of an acetonitrile solution containing 3.55 g (0.0199 M) of N-bromosuccinimide were added dropwise to this solution with ice cooling. After stirring at the same temperature for 30 minutes, 1.6 ml (0.0198 M) of pyridine was added dropwise over a period of 10 minutes. The solution was stirred at room temperature for 10 minutes and poured into 100 ml of ice water; crystals were formed. The crystals were filtered and purified by silica gel column chromatography. The purified crystals were recrystallized from ethyl acetate to give 1.28 g of 2-dodecyl-7,7,8,8-tetracyanoquinodimethane, in the form of yellow acicular crystals.

This compound had the following characteristics:
Melting point: 120°C
Infrared absorption band: 2250 $cm^{-1}$ (cyano)
Calculated for $C_{24}H_{28}N_4$: C 77.38%; H 7.58%; N 15.04%
Found: C 77.48%; H 7.87%; N 15.08%

EXAMPLE 2

12 Grams of 2-pentadecyl-hydroquinone were dissolved in 100 ml of methanol and the solution was hydrogenated in a hydrogen atmosphere at an initial pressure of 130 $kg/cm^2$ in the presence of Raney nickel as a catalyst at 170°C. After cooling, the catalyst was removed and the solvent was evaporated to give 2-pentadecyl-1,4-cyclohexane-diol crystals. 8.7% of these crystals were dissolved in 200 ml of methylene chloride containing 26.6 g (0.001 M) of a complex of pyridinium chlorochromate and alumina and the solution was stirred for 2 hours with ice cooling. After filtering off the oxidizing agent and evaporating the solvent, 5.6 g of 2-pentadecyl-1,4-cyclohexanedione, in the form of white scale-like crystals, were obtained after purification by silica gel column chromatography.

The thus obtained compound had a melting point of 87°C and an IR absorption band of 1710 $cm^{-1}$ which corresponds to a carbonyl group.

Steps (b) and (c) of Example 1 were repeated using the 2-pentadecyl-1,4-cyclohexanedione so obtained as a starting material, whereby 1,4-bis(dicyanomethylene)-2-pentadecyl cyclohexane, m.p. 98°C, and 2-pentadecyl-7,7,8,8-tetracyanoquinodimethane were obtained. The yield of the 1,4-bis-(dicyanomethylene)-2-pentadecyl cyclohexane and 2-pentadecyl-7,7,8,8,-tetracyanoquinodimethane were 72% and 46.2% respectively. The thus obtained 2-pentadecyl-TCNQ had a melting point of 120.5°C and an IR absorption band of 2250 $cm^{-1}$ which corresponds to a cyano group.

EXAMPLE 3

The process of Example 2 was repeated except that 2-octadecyl-hydroquinone was used instead of 2-pentadecyl hydroquinone. The intermediates and the final product were as follows:
2-octadecyl-1,4-cyclohexanedione (m.p. 87.5°C; yield 58.5%)
1,4-bis-(dicyanomethylene)-2-octadecyl-cyclohexane (m.p. 99°C; yield 57%)
2-octadecyl-7, 7, 8, 8, -tetracyanoquinodimethane (yield 38.6%)
The 2-octadecyl-TCNQ thus obtained had a melting point of 125.5°C and an infrared absorption band of 2250 $cm^{-1}$ which corresponds to a cyano group.

Formation of monomolecular film by the Langmuir-Blodgett technique.

2-Dodecyl-TCNQ obtained by the process of Example 1 was dissolved in chloroform to make a $10^{-4}$ M solution. This solution was added dropwise to the surface of an aqueous solution containing 4 x $10^{-4}$ mol of cadmium chloride and 5 x $10^{-4}$ mol of potassium hydrogen carbonate and having a pH of 5.5. Upon evaporation of the chloroform, the dodecyl-TCNQ was spread on the surface of the aqueous solution in the form of a monomolecular layer or film. A series of monomolecular layers could be built upon this first layer.

**Claims**

1. 7,7,8,8-Tetracyanoquinodimethane derivatives of the formula:

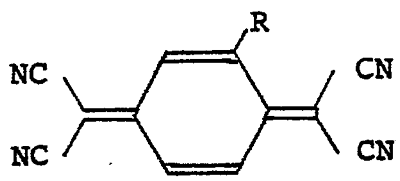

in which R is an alkyl group having from 10 to 22 carbon atoms.

2. A charge transfer complex which comprises a 7,7,8,8-tetracyanoquinodimethane derivative as specified in claim 1, as an electron acceptor, and at least one of tetrathiofulvalene, heterocyclic quaternary ammonium salts and cyanine dyes, as an electron acceptor.

3. A method of preparing a 7,7,8,8-tetracyanoquinodimethane derivative as specified in claim 1, which comprises hydrogenating a hydroquinone derivative containing an alkyl substituent having from 10 to 22 carbon atoms to form a corresponding alkyl-1,4-cyclohexanedione derivative, reacting the latter with malononitrile to form a corresponding alkyl-tetracyanocyclohexanedimethylidene derivative, and oxidising the latter to form the desired compound.

**Patentansprüche**

1. 7,7,8,8-Tetracyanochinodimethan-Derivate der Formel

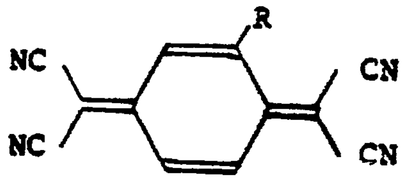

worin R eine Alkylgruppe mit 10 bis 22 Kohlenstoffatomen ist.

2. Ladungsübertragungskomplex, der ein 7,7,8,8-Tetracyanochinodimethan-Derivat nach Anspruch 1 als Elektronenakzeptor und wenigstens eine Verbindung aus Tetrathiofulvalen, heterocyclischen quaternären Ammoniumsalzen und Cyaninfarbstoffen als Elektronenakzeptor umfaßt.

3. Verfahren zur Herstellung eines 7,7,8,8-Tetracyanochinodimethan-Derivats nach Anspruch 1, bei dem ein Hydrochinonderivat, enthaltend einen Alkylsubstituenten mit 10 bis 22 Kohlenstoffatomen, hydriert wird zur Bildung eines entsprechenden Alkyl-1-4-cyclohexandion-Derivats, das letztere mit Malonsäuredinitril zur Bildung eines entsprechenden Alkyl-tetracyanocyclohexandimethyliden-Derivats umgesetzt wird und das letztere oxidiert wird zur Bildung der gewünschten Verbindung.

**Revendications**

1. Dérivés du 7,7,8,8-tétracyanoquinodiméthane, de formule:

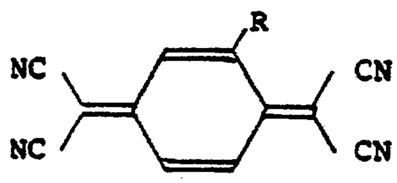

dans laquelle R représente un groupe alkyle ayant de 10 à 22 atomes de cabone.

2. Complexe à rôle de transfert de charges, qui comprend un dérivé de 7,7,8,8-tétracyanoquinodiméthane, tel que spécifié à la revendication 1, comme accepteur d'électrons, et au moins un composé choisi parmi du tétrathiofulvalène, des sels d'ammoniums quaternaires hétérocycliques et des colorants de la famille de la cyanine, comme accepteur d'électrons.

3. Procédé pour préparer un dérivé de 7,7,8,8-tétracyanoquinodiméthane, tel que spécifié à la revendication 1, qui comprend l'hydrogénation d'un dérivé de l'hydroquinone, contenant un substituant alkyle ayant de 10 à 22 atomes de carbone, pour former un dérivé correspondant d'alkyl-1,4-cyclohexanedione, la réaction de ce dernier avec le malononitrile pour former un dérivé correspondant d'alkyl-tétracyanocyclohexanediméthylidène et l'oxydation de ce dernier pour former le composé voulu.